(19) Europäisches Patentamt
European Patent Office
Office européen des brevets

(11) Publication number: 0 186 915 B1

(12) **EUROPEAN PATENT SPECIFICATION**

(45) Date of publication of patent specification: **08.08.90**

(51) Int. Cl.$^5$: **C 07 D 307/52**, A 61 K 31/34

(21) Application number: **85116657.9**

(22) Date of filing: **30.12.85**

(54) **Novel allylic amines.**

(30) Priority: **31.12.84 US 687627**

(43) Date of publication of application:
**09.07.86 Bulletin 86/28**

(45) Publication of the grant of the patent:
**08.08.90 Bulletin 90/32**

(84) Designated Contracting States:
**AT BE CH DE FR GB IT LI LU NL SE**

(56) References cited:
**EP-A-0 067 105**

**ARZNEIMITTEL-FORSCHUNG, vol. 12, no. 9, September 1962, pages 902-907, Aulendorf, DE; J.B. VAN DER SCHOOT et al.: "Phenylisopropylamine derivatives, structure and action"**

**The file contains technical information submitted after the application was filed and not included in this specification**

(73) Proprietor: **MERRELL DOW PHARMACEUTICALS INC.**
**2110 East Galbraith Road**
**Cincinnati Ohio 45215-6300 (US)**

(72) Inventor: **Bargar, Thomas M.**
**125 Lynn Court**
**Zionsville Indiana 46077 (US)**
Inventor: **Broersma, Robert, Jr.**
**1233 Willow Way**
**Noblesville Indiana 46060 (US)**
Inventor: **McCarthy, James R.**
**1100 Maxwell Lane**
**Zionsville Indiana 46077 (US)**

(74) Representative: **Sgarbi, Renato et al**
**GRUPPO LEPETIT S.p.A. Patent and Trademark Department 34, Via Roberto Lepetit**
**I-21040 Gerenzano (Varese) (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 186 915 B1

## Description

This invention relates to novel allylic amines, the processes and intermediates useful for their preparation, and to the pharmaceutical compositions and the method of treating hypertension with such compositions.

More specifically, this invention relates to allylic amines of the formula

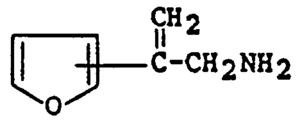

I

and the non-toxic pharmaceutically acceptable acid addition salts thereof. Still more specifically, the compounds of this invention relate to β-methylene-2-furanethanamine and β-methylene-3-furanethanamine and the non-toxic pharmaceutically acceptable acid addition salts thereof.

A class of alkyl amine MAO inhibitors is described in EP—A—67105.

Representative salts are those salts formed with non-toxic organic or inorganic acids, such as, for example those formed from the following acids: hydrochloric, hydrobromic, sulfonic, sulfuric, phosphoric, nitric, maleic, fumaric, benzoic, ascorbic, succinic, methanesulfonic, acetic, propionic, tartaric, citric, lactic, malic, mandelic, cinnamic, palmitic, itaconic, and benzenesulfonic.

The allylic amines (I) of this invention can readily be prepared by a series of reactions illustrated by the following reaction scheme:

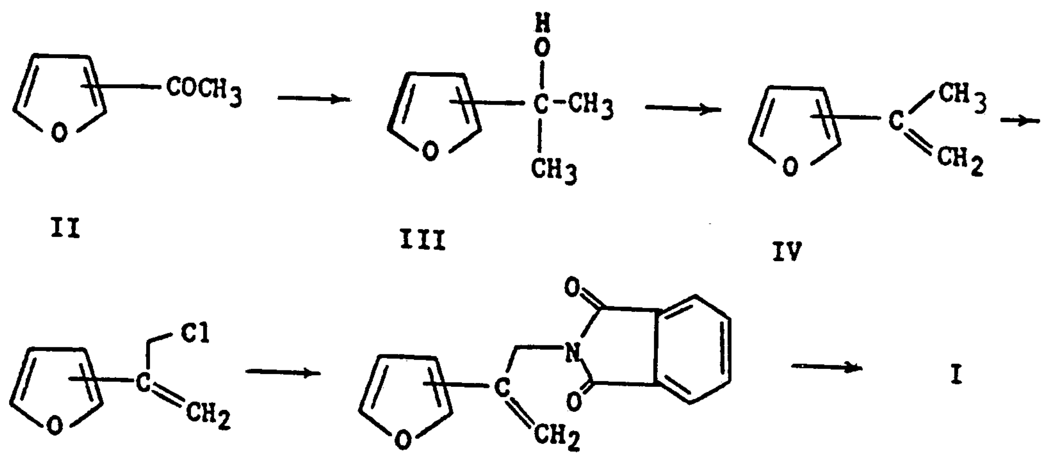

II III IV

V VI I

In essence, the foregoing reaction scheme depicts the conversion of 2- or 3-acetyl derivatives of furan to the corresponding 2- or 3-isopropylidene derivatives by reactions with methylmagnesium bromide with subsequent dehydration according to standard Grignard reaction conditions. The isopropylidene derivatives (IV) are subjected to allylic chlorination according to standard conditions and the crude products (V) are converted (via phthalimide derivatives (VI)) by the well-known Gabriel synthesis to obtain the desired allylic amines of formula I. The free bases can be converted to the acid addition salts, or the acid addition salts can be converted to the free bases, by conventional chemical methodology.

The foregoing reaction scheme is further illustrated by the following specific exemplifications.

### Example 1

β-Methylene-2-Furanethanamine Hydrochloride

*Step A: 2-(1-Methyl)ethenylfuran:*

A solution of 55.06 g (0.5 mole) of 2-acetylfuran in 100 ml of anhydrous ether was added dropwise under $N_2$ during 1.5 hour to 211 ml of 2.85 M methylmagnesium bromide/ether (0.6 mole) while the reaction mixture was stirred in an ice bath. The temperature was kept below 30°C by controlling the rate of addition. A grey precipitate formed. The mixture was allowed to warm to 25°C for 1 hour, then was cooled again in an ice bath while 100 ml of saturated $NaHCO_3$ solution was added carefully. The resulting mass was dissolved in about 1 liter of water and the aqueous phase was extracted twice with ether. The combined ether solutions were extracted with saturated NaCl solution, dried over $K_2CO_3$, filtered, and concentrated at atmospheric pressure to a yellow oil. To this crude alcohol was added 5.0 g of $KHSO_4$ and about 0.1 g of 4-*tert*-butyl catechol (inhibitor) and the mixture was distilled at 1 atm. A mixture of the

desired product and water distilled over at about 90°C. The water was separated and the product was dried over $K_2CO_3$, then filtered to afford 8.5 g of colorless oil. Similarly prepared is 3-(1-methyl)ethenylfuran.

*Step B: N-2-(2-Furanyl)propenylphthalimide:*

To a solution of 8.35 g (0.077 mole) of the olefin of Step A in 310 ml of DMF was added 12.37 g (0.093 mole) of N-chlorosuccinimide and 1.46 g (0.0047 mole) of diphenyl diselenide. After 3 hours at room temperature the mixture was partitioned between 500 ml of hexane and 1000 ml of 5% $Na_2S_2O_3$. The hexane was distilled off at atmospheric pressure and the residue was dissolved in 200 ml of DMF, 9.49 g (0.051 mole) of potassium phthalimide was added and the mixture was warmed to 90°C under $N_2$. After 45 min. the cooled reaction mixture was poured into water and the precipitated product was filtered off and recrystallized from ethylacetate/2-propanol, affording 3.57 g colorless crystals, mp. 136—137°C.

*Anal.*

| | | | |
|---|---|---|---|
| Calc'd for $C_{15}H_{11}NO_2S$: | C, 66.90; | H, 4.12; | N, 5.20. |
| Found: | C, 66.82; | H, 4.30; | N, 4.97. |

Similarly prepared is N-2-(3-furanyl)propenylphthalimide.

*Step C: β-Methylene-2-furanethanamine HCL:*

To a magnetically stirred suspension of 3.50 g (13.83 mmol) of the phthalimide prepared in Step B was added 1.34 ml (27.64 mmol) of hydrazine hydrate and 400 ml of ethanol and the mixture was refluxed under $N_2$ for 1 hour, during which time a thick precipitate formed. The cooled mixture was distilled with 1 M KOH to dissolve the precipitate, then was extracted with ether. The ether layer was washed with 1 M KOH, then was extracted with 1 M HCl. The acid layer was made basic with 5 N NaOH, then was saturated with NaCl and extracted with ether. The ether phase was dried over $K_2CO_3$ and concentrated to a yellow oil. Distillation afforded 1.23 g of colorless liquid, bp 40°C at 0.5 torr. The amine was taken up in ether, cooled in an ice bath, and a saturated solution of anhydrous HCl in ether was added dropwise until no more precipitate formed. The volatiles were removed under vacuum and the residue was recrystallized from ethanol/ethyl acetate to afford 1.2 g colorless crystals, mp 150—151°C.

*Anal.*

| | | | |
|---|---|---|---|
| Calc'd for $C_7H_9NO \cdot HCl$: | C, 52,68; | H, 6.31; | N, 8.78. |
| Found: | C, 52.48; | H, 6.43; | N, 8.61. |

Similarly prepared is β-methylene-3-furanethanamine·HCl.

The allylic amines of this invention (I) are dopamine β-hydroxylase (DBH) inhibitors in a mechanism-based fashion; inactivation being time, and concentration dependent. Thus the compounds of formula I are expected to be valuable therapeutic agents useful in the treatment of hypertension.

The dopamine β-hydroxylase inhibitory properties of the compounds of this invention can readily be determined by standard and well known procedures such as those procedures set forth in U.S. Patent 4,415,191. For example, determination of whether the DBH inhibition allows time-dependent kinetics is exemplified by the procedure wherein enzymatic oxygenation by DBH is determined in aqueous solution in the presence of molecular oxygen, an electron donor such as ascorbate, and the necessary cofactors for the enzyme at a pH of 5 and a temperature of 20°—40°C, preferably 37°C. The test compound is added at the desired concentration, and the system is incubated. At different time intervals, aliquots are taken and DBH activity is measured using tyramine as the substrate and the reaction followed by measuring the oxygen uptake using a polarographic electrode and an oxygen monitor by the method of S. May et al., J. Biol. Chem. 256, 2258 (1981). The inhibition constants for the inactivation of DBH by each compound are then determined by conventional procedures such as the method of Kitz and Wilson, J. Biol. Chem. 237, 3245 (1962). When the compound shown in Table I was tested according to the above described procedure, the DBH inhibitory activity increased as a function of the time of incubation. The initial rate of decrease of activity increased with increasing concentration of inhibitor. The results in Table I indicate that α-methylene-2-furanethanamine is potent as illustrated by the rapid rate of inactivation ($k_{inact}$) and low inhibition constant ($K_I$).

Table I
DBH Inhibitory Activity — In Vitro

| Compound | $K_I$ (mM) | $K_{inact.}$ ($min^{-1}$) |
|---|---|---|
| β-Methylene-2-Furanethanamine | 8 | 0.004 |

Table II
Antihypertensive Activity — In Vivo

| Compound | Dose mg/kg | Maximum % Change Mean Blood Pressure |
|---|---|---|
| β-Methylene-2-Furanethanamine | 10 (ip) | 18 |
| | 30 (ip) | 32 |

The ability of the compounds of this invention to lower blood pressure can be determined *in vivo* using hypertensive rats according to standard and well known procedures. The test compound is administered interperitoneally (ip) or orally (po) to rats and the blood pressure monitored continuously. Since DBH is a major enzyme in the synthetic pathway of the catecholamines, it would be expected that the presence of an inhibitor would act to decrease the amount of catecholamines produced, and thereby have an antihypertensive effect. The results of the testing for this antihypertensive effect are shown in Table II.

Thus, based upon these and other standard laboratory techniques known to evaluate dopamine β-hydroxylase inhibitors, by standard toxicity tests and by standard pharmacological assay for the determination of anti-hypertensive activity in mammals, and by comparison of these results with the results with known antihypertensive agents, the effective antihypertensive dosage of the compounds of this invention can readily be determined. In general, effective antihypertensive results can be achieved at a dose of about 5 to about 100 mg per kilogram of body weight per day. Of course the specific initial and continuing dosage regimen for each patient will vary according to the nature and severity of the hypertension as determined by the attending diagnostician.

In their function as therapeutically useful compounds, it is advantageous to administer the compounds to the host animal in admixture with an acceptable pharmaceutical carrier suitable for enteral or parenteral administration, said carrier constituting a major portion of the admixture. Such preparations may be in such forms as, for example, tablets, capsules and suppositories, or in liquid forms, as for example, elixirs, emulsions, sprays and injectables. In the formulation of pharmaceutical preparations there can be employed such substances which do not react with active substances as, for example, water, gelatin, lactose, starches, magnesium sterate, talc, vegetable oils, benzyl alcohols, gums, polyalkylene glycols, petroleum jelly and the like. The active ingredient of such pharmaceutical preparations is preferably present in the preparation in such proportions by weight that the proportion by weight of the active ingredient to be administered lies between 0.1% and 50%.

**Claims for the Contracting States: BE CH DE FR GB IT LU NL SE**

1. A compound of the formula

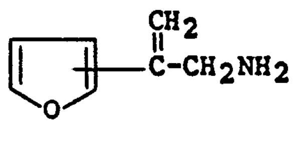

I

and the non-toxic pharmaceutically acceptable acid addition salts thereof.

2. A compound of claim 1, said compound being β-methylene-2-furanethanamine.

3. A compound of claim 1, said compound being β-methylene-3-furanethanamine.

4. A compound of claim 1 for use as a medicine.

5. Use of a compound of claim 1 for preparing a medicament for treating hypertension.

6. A pharmaceutical composition which comprises a compound of claim 1 in admixture with a pharmaceutically acceptable carrier.

7. A process for preparing an allylic amine of the formula

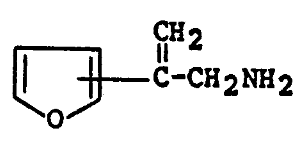

I

which comprises hydrolyzing a compound of the formula

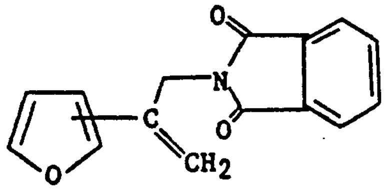 II

8. A process according to claim 7 wherein the hydrolysis is effected by heating a compound of formula II with hydrazine in the presence of an alcohol.

**Claims for the Contracting State: AT**

1. A process for preparing an allylic amine of the formula

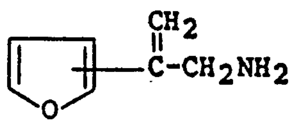 I

which comprises hydrolyzing a compound of the formula

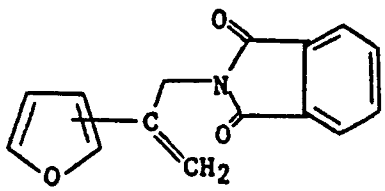 II

2. A process according to claim 1 wherein the hydrolysis is effected by heating a compound of formula II with hydrazine in the presence of an alcohol.

3. A process for preparing a compound of claim 1, said compound being β-methylene-2-furanethanamine.

4. A process for preparing a compound of claim 1, said compound being β-methylene-3-furanethanamine.

5. Use of a compound of claim 1 for preparing a medicament for combatting hypertension.

**Patentansprüche für die Vertragsstaaten: BE CH DE FR GB IT LU NL SE**

1. Eine Verbindung der Formel

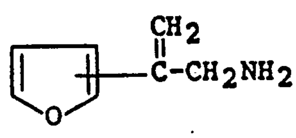 I

und die nicht-toxischen, pharmazeutisch verträglichen Säureadditionssalze davon.

2. Verbindung nach Anspruch 1, nämlich β-Methylen-2-furanethanamin.

3. Verbindung nach Anspruch 1, nämlich β-Methylen-3-furanethanamin.

4. Verbindung nach Anspruch 1 zur Verwendung als Arzneistoff.

5. Verwendung einer Verbindung nach Anspruch 1 zur Herstellung eines Arzneimittels zur Behandlung von Bluthochdruck.

6. Arzneimittel, umfassend eine Verbindung nach Anspruch 1 im Gemisch mit einem pharmazeutisch verträglichen Träger.

7. Verfahren zur Herstellung eines Allylamins der Formel

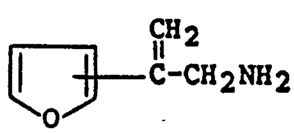 I

umfassend die Hydrolyse einer Verbindung der Formel

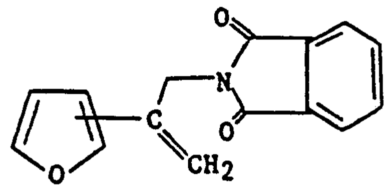

II

8. Verfahren nach Anspruch 7, wobei die Hydrolyse durch Erhitzen einer Verbindung der Formel II mit Hydrazin in Gegenwart eines Alkohols durchgeführt wird.

**Patentansprüche für den Vertragsstaat: AT**

1. Verfahren zur Herstellung eines Allylamins der Formel

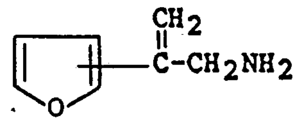

I

umfassend die Hydrolyse einer Verbindung der Formel

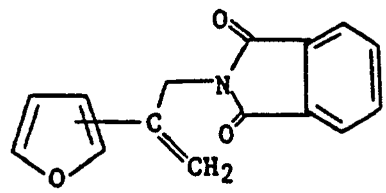

II

2. Verfahren nach Anspruch 1, wobei die Hydrolyse durch Erhitzen einer Verbindung der Formel II mit Hydrazin in Gegenwart eines Alkohols durchgeführt wird.

3. Verfahren zur Herstellung einer Verbindung nach Anspruch 1, wobei die Verbindung β-Methylen-2-furanethanamin ist.

4. Verfahren zur Herstellung einer Verbindung nach Anspruch 1, wobei die Verbindung β-Methylen-3-furanethanamin ist.

5. Verwendung einer Verbindung nach Anspruch 1 zur Herstellung eines Arzneimittels zur Bekämpfung von Bluthochdruck.

**Revendications pour les Etats Contractants: BE CH DE FR GB IT LU NL SE**

1. Un composé de formule

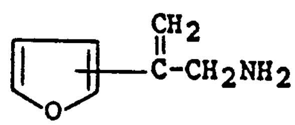

I

et ses sels d'addition d'acides non toxiques acceptables en pharmacie.

2. Un composé selon la revendication 1, ledit composé étant la β-méthylène-2-furannéthanamine.

3. Un composé selon la revendication 1, ledit composé étant la β-méthylène-3-furannéthanamine.

4. Un composé selon la revendication 1, pour l'utilisation comme médicament.

5. Utilisation d'un composé selon la revendication 1 pour préparer un médicamment pour traiter l'hypertension.

6. Une composition pharmaceutique qui comprend un composé selon la revendication 1 en mélange avec un support acceptable en pharmacie.

7. Procédé pour fabriquer une amine allylique de formule

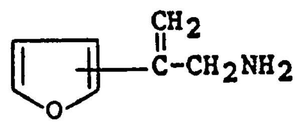

I

qui consiste à hydrolyser un composé de formule

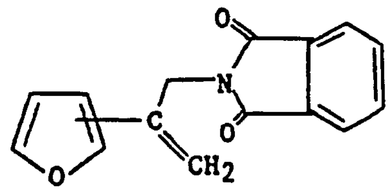

II

8. Procédé selon la revendication 7 dans lequel l'hydrolyse est effectué en chauffant un composé de formule II avec l'hydrazine en présence d'un alcool.

**Revendications pour l'Etat Contractant: AT**

1. Un procédé pour fabriquer une amine allylique de formule

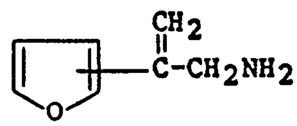

I

qui consiste à hydrolyser un composé de formule

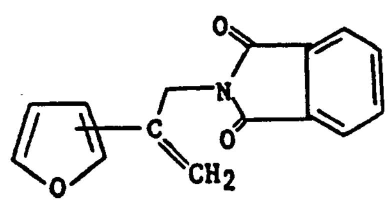

II

2. Procédé selon la revendication 1 dans lequel l'hydrolyse est effectuée en chauffant un composé de formule II avec l'hydrazine en présence d'un alcool.

3. Un procédé pour fabriquer un composé selon la revendication 1, ledit composé étant la β-méthylène-2-furannéthanamine.

4. Un procédé pour fabriquer un composé selon la revendication 1, ledit composé étant la β-méthylène-3-furannéthanamine.

5. Utilisation d'un composé selon la revendication 1 pour fabriquer un médicament pour combattre l'hypertension.